(19) Europäisches Patentamt
European Patent Office
Office européen des brevets

(11) Numéro de publication: **0 586 648 B1**

(12) # FASCICULE DE BREVET EUROPEEN

(45) Date de publication du fascicule du brevet: **06.12.95**

(51) Int. Cl.$^{6}$: **C07C 323/35**, C07C 323/41, A61K 7/13

(21) Numéro de dépôt: **93905390.6**

(22) Date de dépôt: **12.02.93**

(86) Numéro de dépôt internationale :
**PCT/FR93/00148**

(87) Numéro de publication internationale :
**WO 93/16990 (02.09.93 93/21)**

(54) **COMPOSITION TINCTORIALE POUR FIBRES KERATINIOUES CONTENANT DES METAPHENYLENEDIAMINES SOUFREES, PROCEDE DE TEINTURE ET NOUVELLES METAPHENYLENEDIAMINES SOUFREES ET LEUR PROCEDE DE PREPARATION.**

(30) Priorité: **14.02.92 FR 9201704**
**14.02.92 FR 9201705**

(43) Date de publication de la demande:
**16.03.94 Bulletin 94/11**

(45) Mention de la délivrance du brevet:
**06.12.95 Bulletin 95/49**

(84) Etats contractants désignés:
**DE ES FR GB IT**

(56) Documents cités:
**EP-A- 0 303 826**
**EP-A- 0 331 144**
**DE-A- 3 343 642**
**DE-A- 3 724 642**
**FR-A- 2 362 116**

(73) Titulaire: **L'OREAL**
**14, rue Royale**
**F-75008 Paris (FR)**

(72) Inventeur: **JUNINO, Alex**
**16, rue Docteur-Bergonié**
**F-93190 Livry-Gargan (FR)**
Inventeur: **GENET, Alain**
**9, rue des Coquelicots**
**F-93600 Aulnay-sous-Bois (FR)**
Inventeur: **LAGRANGE, Alain**
**29, rue Auguste-Renoir**
**F-78400 Chatou (FR)**

(74) Mandataire: **Casalonga, Axel et al**
**BUREAU D.A. CASALONGA - JOSSE**
**Morassistrasse 8**
**D-80469 München (DE)**

EP 0 586 648 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouvelles compositions tinctoriales pour fibres kératiniques contenant des métaphénylènediamines soufrées, à un procédé de teinture en milieu alcalin mettant en oeuvre ces compositions, à de nouvelles métaphénylènediamines soufrées ainsi qu'a leur procédé de préparation.

On a déjà utilisé des dérivés soufrés d'amines aromatiques. associés à des précurseurs de colorants d'oxydation, en présence d'agent oxydant et de sels alcalins en milieu acide, neutre ou basique, pour la teinture de fibres kératiniques. De telles compositions sont décrites dans le brevet DE 593 061.

Il est connu de teindre les fibres kératiniques et en particulier les cheveux humains, avec des compositions tinctoriales contenant des précurseurs de colorants d'oxydation et des coupleurs. Le DE-A- 3 343 642 décrit de telles compositions contenant en tant que coupleur un 6-hydroxyalkylthio 1, 3-diaminobenzène.

Les coupleurs, encore appelés modificateurs de coloration, permettent de faire varier les nuances obtenues avec les précurseurs de colorants d'oxydation.

Dans le domaine de la teinture des fibres kératiniques et en particulier des cheveux humains, on est à la recherche de coupleurs qui, associés à des précurseurs de colorants d'oxydation, permettent d'obtenir un large éventail de nuances, tout en conférant aux cheveux une coloration ayant une résistance satisfaisante à la lumière, au lavage, aux intempéries, à la transpiration et aux différents traitements que peuvent subir les cheveux.

La demanderesse vient de découvrir, ce qui fait l'objet de l'invention, que l'utilisation de métaphénylènediamines soufrées, de formule (I) à titre de coupleur avec des précurseurs de colorants d'oxydation de type ortho et/ou para permettait d'obtenir, à pH neutre, acide ou alcalin, après application sur les fibres kératiniques et en particulier les cheveux humains, un large éventail de nuances de coloration présentant une résistance à la lumière, au lavage, aux intempéries, à la transpiration et aux différents traitements que peuvent subir les cheveux, particulièrement remarquable.

Un objet de l'invention est donc constitué par des compositions tinctoriales d'oxydation, destinées à être utilisées pour la teinture des fibres kératiniques et en particulier des cheveux humains, contenant au moins un précurseur de colorant d'oxydation de type ortho et/ou para et au moins une métaphénylènediamine soufrée de formule (I) ci-après.

Un autre objet de l'invention porte sur le procédé de coloration des fibres kératiniques et en particulier des cheveux humains, mettant en oeuvre une telle composition mélangée à un agent oxydant.

L'invention a également pour objet de nouvelles métaphénylènediamines soufrées ainsi que leur procédé de préparation et leur utilisation dans des compositions tinctoriales pour fibres kératiniques.

D'autres objets de l'invention apparaîtront à la lecture de la description et des exemples qui suivent.

La présente invention a donc pour objet les compositions tinctoriales pour fibres kératiniques et en particulier les cheveux humains, contenant, dans un milieu approprié pour la teinture, au moins un précurseur de colorant d'oxydation de type ortho et/ou para, et au moins, à titre de coupleur, une métaphénylènediamine soufrée de formule :

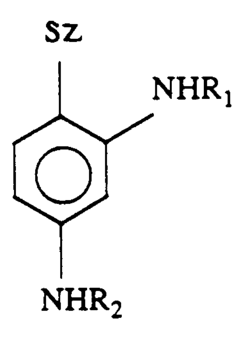

(I)

dans laquelle : Z représente un radical alkyle en $C_1$-$C_{18}$, un radical aralkyle dans lequel le radical alkyle est en $C_1$-$C_6$, un radical monohydroxyalkyle en $C_1$-$C_6$ ou polyhydroxyalkyle $C_2$-$C_6$, un radical aryle, un radical aminoalkyle de formule :

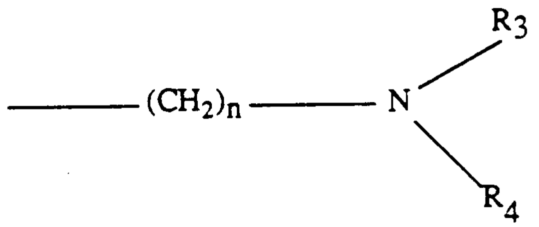

(II)

dans laquelle n est un nombre entier compris entre 1 et 6 inclus, $R_3$ et $R_4$ identiques ou différents représentent un atome d'hydrogène ou un radical alkyle en $C_1$-$C_4$, hydroxyalkyle en $C_1$-$C_4$, acyle en $C_2$-$C_6$; $R_1$ et $R_2$ identiques ou différents représentent un atome d'hydrogène, un radical alkyle en $C_1$-$C_6$, un radical monohydroxyalkyle en $C_1$-$C_6$, polyhydroxyalkyle en $C_2$-$C_6$, monocarbamylalkyle en $C_1$-$C_6$, dicarbamylalkyle en $C_1$-$C_6$, aminoalkyle en $C_1$-$C_6$, acyle en $C_2$-$C_6$, carbalcoxy en $C_2$-$C_6$, carbamyle ou monoalkyle en $C_1$-$C_6$ carbamyle, avec la condition que $R_1$ et $R_2$ ne désignent pas simultanément un atome d'hydrogène lorsque Z désigne un radical alkyle ou hydroxyalkyle, ainsi que les sels d'acide correspondants aux composés de formule (I).

Parmi les significations préférées du radical Z dans les métaphénylènediamines soufrées de formule générale (I) selon l'invention, le radical alkyle en $C_1$-$C_{18}$ désigne les radicaux méthyle, éthyle, propyle, butyle, dodécyle, hexadécyle; le radical aralkyle désigne le radical benzyle ; le radical mono ou polyhydroxyalkyle désigne $-CH_2-CH_2OH$,
$-CH_2-CHOH-CH_2-OH$, $-CH_2-CHOH-CH_3$; le radical aryle désigne le phényle: le radical aminoalkyle désigne
$-CH_2-CH_2-NH_2$ ;

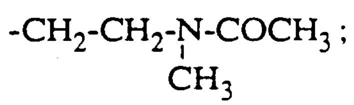

$-CH_2-CH_2-NH\ CH_3$ ;      $-CH_2-CH_2-NHCOCH_3$ ;
lorsque les groupements R représentent un radical acyle, celui-ci, désigne de préférence les radicaux formyle, acétyle et propionyle.

Les sels d'acide correspondant aux composés métaphénylènediamines soufrées de formule générale (I) sont choisis de préférence parmi les chlorhydrates, les sulfates ou les bromhydrates.

Parmi les métaphénylènediamines soufrées de formule générale (I), les composés particulièrement préférés sont :

- le 6-méthylthio 1,3-di(β-hydroxyéthylamino) benzène
- le 6-méthylthio 1-N-β-hydroxyéthylamino 3-amino benzène
- le 6-(β-acétylaminoéthylthio) 1,3 -diaminobenzène
- la 6-méthylthio 3-N-β-hydroxyéthylamino aniline
- la 6-méthylthio 3-acétylamino aniline
- la 6-(β-acétylaminoéthylthio) 3-acétylamino aniline
- le 6-(β-acétylaminoéthylthio) 3-amino 1-acétylaminobenzène et leurs sels.

Les composés de formule (I) sont utilisables comme coupleur en présence de précurseurs de colorants d'oxydation de type ortho et/ou para connus en eux-mêmes, permettant de teindre les cheveux par coloration d'oxydation, selon un processus mettant en oeuvre une réaction de condensation oxydative des précurseurs et du coupleur.

Les précurseurs de colorants de types ortho ou para sont des composés qui ne sont pas des colorants en eux-mêmes, mais qui forment un colorant par un processus de condensation oxydative, soit sur eux-mêmes, soit en présence d'un coupleur ou modificateur.

Ces précurseurs de colorants d'oxydation de type ortho ou para sont des composés benzéniques ou hétérocycliques qui comportent deux groupements fonctionnels amino ou amino et hydroxy, en position ortho ou para l'un par rapport à l'autre.

Les précurseurs de colorants d'oxydation de type ortho ou para peuvent être choisis parmi les paraphénylènediamines, les paraaminophénols, les précurseurs hétérocycliques para dérivés de la pyridine ou de la pyrimidine, tels que la 2,5-diaminopyridine, la 2-hydroxy 5-aminopyridine, la 2,4,5,6-tétraaminopyrimidine, la 4,5-diamino 1-méthylpyrazole, la 2-diméthylamino 4,5,6-triaminopyrimidine, les orthoaminophénols et les bases dites "doubles".

A titre de paraphénylènediamines, on peut plus particulièrement citer les composés répondant à la formule (III) :

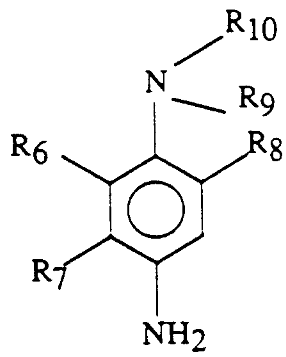

(III)

dans laquelle :

$R_6$, $R_7$, $R_8$ identiques ou différents, représentent un atome d'hydrogène ou d'halogène, un radical alkyle un radical alcoxy, un radical carboxy, sulfo ou hydroxyalkyle en $C_1$-$C_4$ ;

$R_9$ et $R_{10}$, identiques ou différents, représentent un atome d'hydrogène un radical alkyle, hydroxyalkyle alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino, ou bien $R_9$ et $R_{10}$ forment conjointement avec l'atome d'azote auquel ils sont liés, un hétérocycle pipéridino ou morpholino, sous réserve que $R_6$ ou $R_8$ représente un atome d'hydrogène lorsque $R_9$ et $R_{10}$ ne représentent pas un atome d'hydrogène, ainsi que les sels de ces composés. Ces groupes alkyle ou alcoxy ayant de 1 à 4 atomes de carbone et notamment désignant le radical méthyle, éthyle, propyle, méthoxy et éthoxy.

Parmi les composés de formule (III) on peut plus particulièrement citer la paraphénylènediamine, la p-toluylènediamine, la méthoxyparaphénylènediamine, la chloroparaphénylènediamine la 2,3-diméthylparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthyl-aniline, la N,N-di-(β-hydroxyéthyl)-paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl, carbamylméthyl)-aniline, la 3-méthyl 4-amino N,N-(éthyl, carbamylméthyl)aniline, la 4-amino N,N-(éthyl, β-pipéridinoéthyl) aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl,β-morpholinoéthyl)aniline, la 4-amino N,N-(éthyl,β-acétylaminoéthyl) aniline, la 4-amino N-(β-méthoxyéthyl) aniline, la 3-méthyl 4-amino N,N-(éthyl, β-acétylaminoéthyl) aniline, la 4-amino N,N-(éthyl, β-mésylaminoéthyl) aniline, la 3-méthyl 4-amino N,N-(éthyl, β-mésylaminoéthyl) aniline, la 4-amino N,N-(éthyl, β-sulfoéthyl)aniline, la 3-méthyl 4-amino N,N-(éthyl, β-sulfoéthyl) aniline, la N-[(4'-amino)phényl]-morpholine, la N-[-(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine la 2-n-propylparaphénylènediamine,l'hydroxy-2-n-propylparaphénylènediamine, la 2-hydroxyméthylparaphénylènediamine, la N,Ndiméthyl 3-méthyl paraphénylènediamine la N,N-(éthyl, β-hydroxyéthyl)paraphénylènediamine, la N-(dihydroxypropyl)paraphénylènediamine, la N-4'-aminophénylparaphénylènediamine, la N-phénylparaphénylènediamine.

Ces paraphénylènediamines peuvent être introduites dans la composition tinctoriale, soit sous forme de base libre, soit sous forme de sels, tels que chlorhydrate, bromhydrate ou sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 3-(β-hydroxyéthoxy)4-aminophénol, le 2-méthoxyméthyl 4-aminophénol, le 2-aminométhyl 4-aminophénol, le 2-β-hydroxyéthylaminométhyl 4-aminophénol, le 2-éthoxyméthyl 4-aminophénol, le 2-(β-hydroxyéthoxy)méthyl 4-aminophénol.

Les bases dites "doubles"sont des bis-phénylalkylènediamines, répondant à la formule :

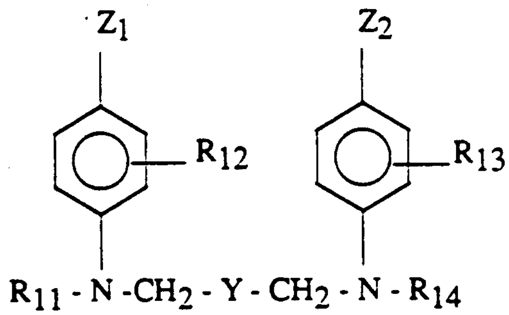

(IV)

dans laquelle :

$Z_1$ et $Z_2$, identiques ou différents, représentent des groupements hydroxyle ou $NHR_{15}$, où $R_{15}$ désigne un atome d'hydrogène ou un radical alkyle inférieur ;

$R_{12}$ et $R_{13}$, identiques ou différents, représentent soit des atomes d'hydrogène, soit des atomes d'halogène, soit encore des radicaux alkyle;

$R_{11}$ et $R_{14}$, identiques ou différents, représentent un atome d'hydrogène, un radical alkyle, hydroxyalkyle ou aminoalkyle, dont le reste amino peut être substitué; Y représente un radical pris dans le groupe constitué par les radicaux suivants :

$-(CH_2)_n-$, $-(CH_2)_m-O-(CH_2)_m-$,

$-(CH_2)_q-CHOH-(CH_2)_q-$,

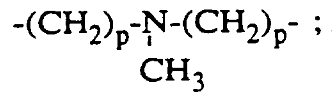

dans lesquels n est un nombre entier compris entre 0 et 8 et m, q et p sont des nombres entiers compris entre 0 et 4, cette base pouvant se présenter également sous forme de ses sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy et éthoxy.

Parmi les composés de formule (IV) on peut citer le N,N'-bis-($\beta$-hydroxyéthyl)N,N'-bis-(4'-aminophényl) 1,3-diamino 2-propanol, la N,N'-bis-($\beta$-hydroxyéthyl)N,N'-bis-(4'-aminophényl)éthylènediamine, la N,N'-bis-(4-aminophényl)tétraméthylènediamine, la N,N'-bis-($\beta$-hydroxyéthyl) N,N'-bis-(4-aminophényl)-tétraméthylènediamine, la N,N'-bis(4-méthylaminophényl)tétraméthylènediamine, la N,N'-bis-(éthyl) N,N'-bis-(4'-amino 3'-méthylphényl)éthylènediamine.

Parmi les orthoaminophénols on peut citer plus particulièrement le 1-amino-2-hydroxybenzène, le 6-méthyl 1-hydroxy 2-aminobenzène, le 4-méthyl 1-amino 2-hydroxybenzène, le 4-acétylamino 1-amino 2-hydroxybenzène.

Les compositions tinctoriales conforment à l'invention peuvent également contenir en plus du coupleur répondant à la formule (I) définie ci-dessus, d'autres coupleurs connus en eux-mêmes, tels que des métadiphénols, des métaaminophénols, des métaphénylènediamines différentes de celles de formule (I) ci-dessus, des métaacylaminophénols, des métauréidophénols, des métacarbalcoxyaminophénols l'$\alpha$-naphtol des dérivés indoliques, des coupleurs possédant un groupement méthylène actif, tels que les composés $\beta$-cétoniques, les pyrazolones.

Parmi ces coupleurs on peut plus particulièrement citer, le 2, 4-dihydroxyphénoxyéthanol, le 2,4-dihydroxyanisole, le métaaminophénol, le monométhyléther de résorcine, la résorcine, la 2-méthyl-résorcine, le 2-méthyl 5-aminophénol, le 2-méthyl 5-N-($\beta$-hydroxyéthyl) aminophénol, le 2-méthyl 5-N-($\beta$-mésylaminoéthyl)aminophénol, le 2,6-diméthyl 3-aminophénol, la 6-hydroxybenzomorpholine, le 2,4-diaminoanisole, le 2,4-diaminophénoxyéthanol, la 6-aminobenzomorpholine, le [2-N-($\beta$-hydroxyéthyl) amino 4-amino]-phénoxyéthanol, le 2-amino 4-N-($\beta$-hydroxyéthyl) amino anisole, le (2,4-diamino)phényl-$\beta$-$\gamma$-dihydroxypropyléther, la 2,4-diaminophénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chloro résorçinol, la 6-méthoxy 3-hydroxyéthylaminoaniline, le 1-éthoxy 2-bis(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3-aminophénol, le 6-chloro 3-trifluoroéthylaminophénol, et leurs sels.

On peut rajouter à ces compositions, comme cela est bien connu dans l'état de la technique, notamment en vue de nuancer ou d'enrichir en reflet les colorations apportées par les précurseurs de colorants d'oxydation, des colorants directs tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représentent de préférence de 0,3 à 7 % en poids par rapport au poids de la dite composition. La concentration en composés métaphénylènediamines soufrées de formule (I) peut varier entre 0,05 et 3,5 % en poids du poids total de la composition.

Les compositions tinctoriales conformes à l'invention contiennent également dans leur forme de réalisation préférée, des agents tensioactifs anioniques, cationiques, non-ioniques, amphotères ou leurs mélanges. Parmi ces agents tensio-actifs, on peut citer les alkylbenzènesulfonates les alkylnaphtalènesulfonates, les sulfates, les éthersulfates et les sulfonates d'alcools gras, les sels d'ammonium quaternaires, tels que le bromure de triméthylcétylammonium, le bromure de cétylpyridinium, les éthanolamides d'acides gras éventuellement oxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en $C_1$-$C_4$, tels que l'éthanol et l'isopropanol; le glycérol; les glycols ou éthers de glycols comme le 2-butoxyéthanol, l'éthylèneglycol le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite. Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique. Ces agents antioxydants sont présents dans la composition dans des proportions comprises entre 0,05 et 1,5 % en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquide, de crème, de gel ou sous toute autre forme appropriée pour une teinture des fibres kératiniques et notamment des cheveux humains. Ces compositions peuvent être conditionnées en flacons aérosols en présence d'un agent propulseur et former des mousses.

Les compositions tinctoriales conformes à l'invention contenant un précurseur de colorant d'oxydation du type para et/ou ortho et un coupleur de formule (I), sont utilisées suivant un procédé mettant en oeuvre la révélation par un agent oxydant.

Conformément à ce procédé on mélange, au moment de l'emploi, la composition tinctoriale décrite ci-dessus avec une solution oxydante en une quantité suffisante pour pouvoir développer une coloration, puis on applique le mélange obtenu sur les fibres kératiniques et en particulier les cheveux humains.

Le pH de la composition appliquée sur les cheveux varie entre 3 et 11. Il est ajusté à la valeur désirée à l'aide d'agents alcalinisants bien connus dans l'état de la technique, tels que l'ammoniaque, les carbonates alcalins, les alcanolamines comme la mono, la di- et la triéthanolamine, ainsi que leurs dérivés ou les hydroxydes de sodium ou de potassium, ou à l'aide d'agents acidifiants classiques, tels que les acides minéraux ou organiques, tels que les acides chlorhydrique. tartrique, citrique, phosphorique et

sulfonique. La solution oxydante contient à titre d'agent oxydant, l'eau oxygénée, le peroxyde durée, des persels, tels que le persulfate d'ammonium ou des bromates de métaux alcalins. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on les rince, les lave au shampooing, on les rince à nouveau et on les sèche.

Le coupleur de formule (I) définie ci-dessus, peut également être mis en oeuvre dans un procédé à plusieurs étapes, consistant dans l'une des étapes, à appliquer le précurseur de colorant d'oxydation du type ortho et/ou para ou leur mélange et, dans une autre étape, a appliquer une composition tinctoriale contenant le coupleur de formule (I).

L'agent oxydant peut être introduit, juste avant l'application, dans la composition appliquée dans le deuxième temps ou bien être appliqué sur les fibres kératiniques elles-mêmes, dans un troisième temps, les conditions de pose, de pH, de lavage et de séchage étant identiques à celles indiquées ci-dessus.

Un autre objet l'invention est constitué par les nouvelles métaphénylènediamines soufrées de formule (I) ci-dessus, ainsi que leurs sels d'acide.

L'invention a aussi pour objet l'utilisation de ces métaphénylènediamines soufrées dans des compositions tinctoriales pour fibres kératiniques.

Les métaphénylènediamines soufrées de formule (I) ou leurs sels peuvent être préparées selon des procédés en plusieurs étapes.

Selon un premier procédé et dans une première étape, on fait réagir en présence d'une base telle que la potasse ou le carbonate de potassium, le bromo- 2,4-dinitrobenzène sur un thiol de formule (V) :

Z'-SH (V)

dans laquelle Z' représente un radical alkyle en $C_1$-$C_{18}$, aralkyle dans lequel le radical alkyle est en $C_1$-$C_6$, monohydroxyalkyle en $C_1$-$C_6$, polyhydroxyalkyle en $C_2$-$C_6$ ou un groupement de formule (VI)

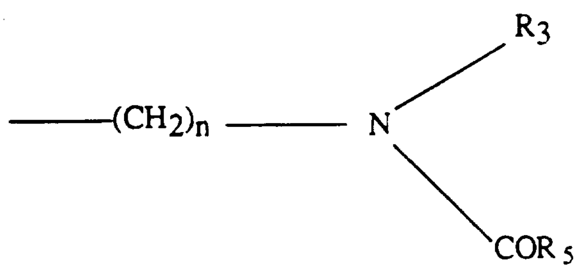

(VI)

dans laquelle $R_3$ et n ont les significations indiquées précédemment dans la formule (I) ; et $R_5$ représente un atome d'hydrogène ou un radical alkyle en $C_1$-$C_3$ ;
dans une deuxième étape, on réduit les substituants nitro du compose de formule (VII) :

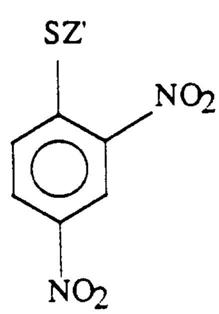

(VII)

obtenu précédemment pour préparer un composé répondant à la formule (VIII) :

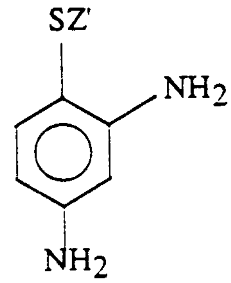

(VIII)

dans laquelle Z' a la signification indiquée ci-dessus ;
éventuellement, dans une troisième étape, et suivant le composé métaphénylènediamine soufrée de formule (I) que l'on souhaite obtenir, on effectue

a) soit une mono-substitution des amines aromatiques pour obtenir un composé de formule (I) dans laquelle $R_1$ et/ou $R_2$ sont différents de H

b) soit une hydrolyse acide du composé (VIII) dans lequel Z' représente

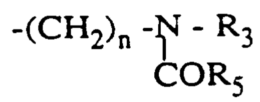

pour obtenir le composé de formule (IX)

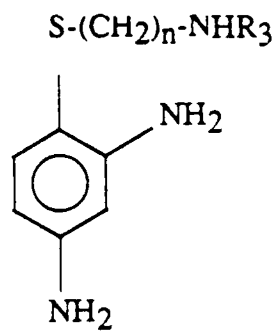

(IX)

dans laquelle $R_3$ et n ont les significations indiquées ci-dessus, $R_3$ ne désignant toutefois pas de radical acyle en $C_2$-$C_6$,
les amines nucléaires pouvant être ensuite monosubstituées.

c) soit on effectue au préalable une substitution de l'amine extranucléaire sur le composé de formule (VIII) pour obtenir le composé de formule (X)

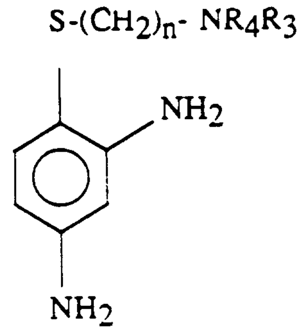

(X)

dans laquelle $R_3$, $R_4$ et n ont les significations indiquées ci-dessus, les amines nucléaires pouvant être ensuite monosubstituées.

La réduction des groupes nitro des composés de formule (VII) s'effectue de préférence en utilisant du fer en milieu acétique ou alors par le cyclohexène en présence d'un catalyseur palladium-charbon ou par tout autre procédé de réduction classique.

La substitution des amines aromatiques ou de l'amine extranucléaire peut être effectuée en faisant réagir, par exemple, le bromure d'éthyle, la bromhydrine du glycol, l'éthyl-chloroformiate, la $\beta$-chloracétamide, ou l'anhydride acétique.

Selon un deuxième procédé et dans une première étape, on fait réagir un fluoro nitro benzène substitué de formules (XI a) ou (XI b)

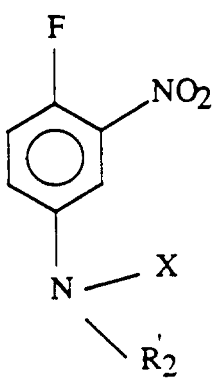

(XI a)

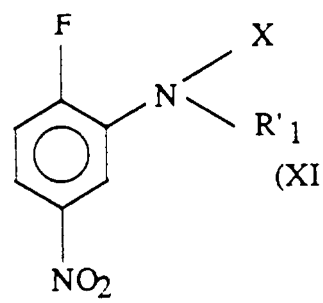

(XI b)

dans lesquelles $R'_1$ et $R'_2$ représentent un groupement acyle en $C_2$-$C_6$ et X représente un atome d'hydrogène, ou bien $R'_2$ et X ou $R'_1$ et X forment, conjointement avec l'atome d'azote auxquels ils sont liés un cycle oxazolidone,
sur un thiol de formule :

Z'-SM (XII)

dans laquelle, M est un alcalin et Z' a les significations indiquées ci-dessus ;
dans une deuxième étape, on réduit le substituant nitro du composé de formules (XIII a) ou (XIII b)

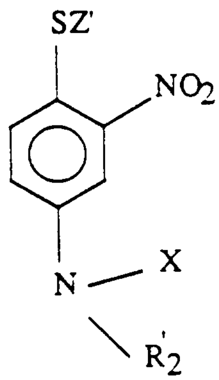

(XIII a)

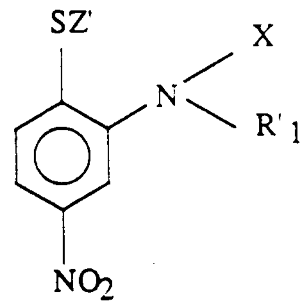

(XIII b)

obtenu précédemment pour préparer un composé répondant à la formule (XIVa) ou (XIVb) :

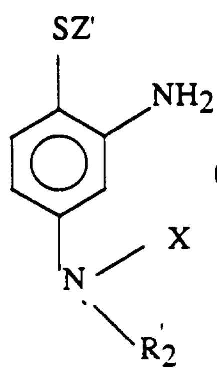

(XIV a)

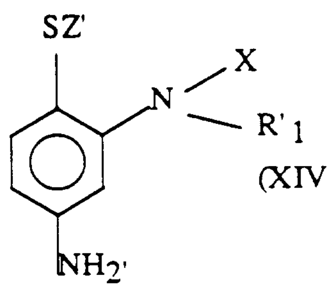

(XIV b)

dans lesquelles $R'_1$, $R'_2$ et X ont les significations indiquées ci-dessus;
éventuellement, dans une troisième étape, et suivant le composé métaphénylènediamine soufrée de formule (I) que l'on souhaite obtenir, on effectue une monosubstitution des amines aromatiques pour obtenir un composé de formule (I) dans laquelle $R_1$ et/ou $R_2$ sont différents de H
Enfin dans une dernière étape, on soumet le composé à une hydrolyse acide afin de couper le groupement protecteur X.

Les exemples qui suivent sont destinés à illustrer l'invention sans pour autant présenter un caractère limitatif.

Exemple 1

Préparation du dichlorhydrate de 6-méthylthio 1,3-di-(β-hydroxyéthylamino) benzène

1ère étape :

Synthèse du 6-méthylthio 1,3-di(β-chlorocarbéthoxy amino) benzène.

A une suspension de 15, 9 g (0, 07 mole) de dichlorhydrate de 6-méthylthio 1, 3-diaminobenzène et de 28 g de carbonate de calcium dans 12 ml de dioxane, chauffée au bain Marie bouillant, on ajoute goutte à goutte 14, 5 ml de β-chloréthylchloroformiate.

Après 30 mn de chauffage on coule cette suspension dans 500 ml d'eau glacée acidifiée avec 60 ml d'une solution d'acide chlorhydrique concentrée.

Une huile cristallise.

On essore, réempate dans l'eau et sèche à 45 °C sur anhydride phosphorique et sous vide.

On obtient des cristaux blancs (24, 4 g) qui, après recristallisation dans l'isopropanol fondent à 119 °C et dont l'analyse élémentaire calculée pour $C_{13}H_{16}N_2O_4SCl_2$ est :

| Analyse | C | H | N | O | S | Cl |
|---|---|---|---|---|---|---|
| Théorie %<br>Trouvé % | 42, 52<br>42, 72 | 4, 39<br>4, 48 | 7, 63<br>7, 61 | 17, 43<br>17, 54 | 8, 73<br>8, 61 | 19, 31<br>19, 24 |

2ème étape :

Hydrolyse alcaline

Le composé obtenu en 1ère étape (22,8 g - 0,062 mole) est mis en suspension dans 54 ml d'éthanol à 96°, 27 ml d'eau et 54 ml de lessive de soude 10 N.

On chauffe au reflux de l'alcool jusqu'à hydrolyse complète (10 mn) de l'oxazolidone intermédiaire.

La réaction est suivie en chromatographie sur couche mince (gel de silice; éluant : acétate d'éthyle).

On évapore l'alcool sous pression réduite, on obtient une huile brune en suspension à laquelle on ajoute 100 ml d'eau glacée et on extrait à l'éther éthylique ( environ 500 ml).

La phase éthérée est séchée sur sulfate de sodium et filtrée. On y ajoute 20 ml d'une solution environ 7 N d'acide chlorhydrique dans l'éthanol absolu.

Le dichlorhydrate précipite en huile qui cristallise.

Après essorage, lavage à l'éther éthylique et séchage sur potasse, on obtient des cristaux blancs (18,4 g) qui fondent avec décomposition à 158-160°C et dont l'analyse élémentaire calculée pour $C_{11}H_{20}N_2O_2SCl_2$ est :

| Analyse | C | H | N | O | S | Cl |
|---|---|---|---|---|---|---|
| Théorie %<br>Trouvé % | 41, 91<br>41, 89 | 6, 39<br>6, 42 | 8, 89<br>8, 90 | 10, 15<br>10, 27 | 10, 17<br>10, 08 | 22, 49<br>22, 58 |

Exemple 2

Préparation du 6-méthylthio 1-N-$\beta$-hydroxyéthylamino 3-amino benzène.

1ère étape.

Synthèse du 6-méthylthio 3-acétylamino nitro-benzène

A température ambiante on met 50,0 g de thiométhylate de sodium en suspension dans 500 ml de diméthoxyéthane et on ajoute, par portions, en 30 mn, 99,1 g (0,5 mole) de 2-nitro 4-acétylamino fluorobenzène en maintenant la température entre 25 et 27°C à l'aide d'un bain d'eau glacée.

Après avoir laissé agiter 30 mn supplémentaires à 25-27°C on coule cette suspension dans 4 litres d'eau glacée.

Le précipité cristallisé jaune-orangé est essoré, réempaté dans l'eau et séché sur anhydride phosphorique.

On obtient 107, 0 g de cristaux orangés qui fondent à 178 °C après recristallisation dans l'éthanol à 96°, et dont l'analyse élémentaire calculée pour $C_9H_{10}N_20_3S$ est :

| Analyse | C | H | N | O | S |
|---|---|---|---|---|---|
| Théorie %<br>Trouvé % | 47, 78<br>47, 90 | 4, 45<br>4, 40 | 12, 38<br>12, 48 | 21, 21<br>21, 01 | 14, 17<br>14, 10 |

2ème étape :

Synthèse du 6-méthylthio 3-acétylamino 1-amino benzène.

On chauffe au reflux de l'alcool une suspension de 0,5 g de chlorure d'ammonium et de 17 g de zinc en poudre fine dans 40 ml d'éthanol à 96° et 3,6 ml d'eau.

On ajoute par portions 6, 8 g (0, 03 mole) du composé obtenu lors de la 1ère étape de façon à maintenir le reflux sans chauffage (on constate une décoloration exothermique).

Après la fin de l'addition, on maintient le reflux pendant 20 mn.

On filtre bouillant et lave les boues de zinc avec le minimum d'alcool chaud.

Le filtrat est dilué avec deux volumes d'eau glacée.

Le composé attendu cristallise lentement.

Après essorage, lavage à l'eau, séchage sur anhydride phosphorique et recristallisation dans l'isopropanol on obtient 2,6 g de cristaux blancs fondant à 105°C et dont l'analyse élémentaire calculée pour $C_9H_{12}N_2OS$ est :

| Analyse | C | H | N | O | S |
|---|---|---|---|---|---|
| Théorie % | 55, 08 | 6, 16 | 14, 27 | 8, 15 | 16, 34 |
| Trouvé % | 55, 07 | 6, 18 | 14, 11 | 8, 32 | 16, 31 |

3ème étape

Synthèse du 6-méthylthio 1-β-chlorocarbéthoxyamino 3-acétylamino benzène.

Ce composé est préparé selon le procédé décrit dans l'exemple 1 (1ère étape).

On obtient des cristaux blancs qui après recristallisation dans l'acétate d'éthyle présentent un point de fusion de 162°C et dont l'analyse élémentaire calculée pour $C_{12}H_{15}N_2O_3SCl$ est :

| Analyse | C | H | N | O | S | Cl |
|---|---|---|---|---|---|---|
| Théorie % | 47, 60 | 4, 99 | 9, 25 | 15, 85 | 10, 59 | 11, 71 |
| Trouvé % | 47, 76 | 5, 07 | 9, 05 | 16, 05 | 10, 43 | 11, 60 |

4ème étape

Hydrolyse alcaline

L'hydrolyse alcaline du composé obtenu à l'étape précédente est faite selon le procédé décrit dans l'exemple 1 (2ème étape).

Après recristallisation de l'acétonitrile, on obtient avec un rendement de 69 % un composé cristallisé blanc dont le point de fusion est 96°C et dont l'analyse élémentaire calculée pour $C_9H_{14}N_2OS$ est :

| Analyse | C | H | N | O | S |
|---|---|---|---|---|---|
| Théorie % | 54, 52 | 7, 12 | 14, 13 | 8, 07 | 16, 17 |
| Trouvé % | 54, 60 | 7, 16 | 14, 11 | 8, 20 | 15, 98 |

Exemple 3

Préparation du dichlorhydrate de 6-méthylthio 3-N-β-hydroxyéthylamino aniline.

1ère étape

Synthèse du 3-(4-méthylsulfanyl-3-nitro-phenyl) -oxazolidin-2-one.

On utilise le procédé décrit pour l'exemple 1 (1ère étape).

A partir de 45,2 g (0,2 mole) de 3-(4-fluoro3-nitro-phenyl)-oxazolidin-2-one on obtient 48,5 g de cristaux orangés qui après recristallisation dans le diméthoxyéthane ont un point de fusion de 180 °C et dont l'analyse élémentaire calculée pour $C_{10}H_{10}N_2O_4S$ est :

| Analyse | C | H | N | O | S |
|---|---|---|---|---|---|
| Théorie %<br>Trouvé % | 47, 24<br>47, 20 | 3, 96<br>3, 99 | 11, 02<br>11, 06 | 25, 17<br>25, 43 | 12, 61<br>12, 49 |

2ème étape :

Synthèse du 6-méthylthio 3-N-β-hydroxyéthylamino nitrobenzène.

L'hydrolyse alcaline du composé précédent est effectuée selon le mode opératoire décrit dans l'exemple 1 (2ème étape).

On obtient avec un rendement de 83 % des cristaux rouges foncés dont le point de fusion est de 104 °C et dont l'analyse élémentaire calculée pour $C_9H_{12}N_2O_3S$ est :

| Analyse | C | H | N | O | S |
|---|---|---|---|---|---|
| Théorie %<br>Trouvé % | 47,36<br>47,34 | 5,30<br>5,38 | 12,27<br>12, 34 | 21,03<br>21,20 | 14,05<br>13,89 |

3ème étape

La réduction du composé précédent est effectuée selon le procédé utilisé pour l'exemple 2 (2ème étape).

Le dichlorhydrate cristallise dans le milieu réactionnel filtré après addition d'éthanol absolu chlorhydrique.

On obtient des cristaux blancs fondant avec décomposition à 154-157 °C et dont l'analyse élémentaire calculée pour $C_9H_{16}N_2OSCl_2$ est :

| Analyse | C | H | N | O | S | Cl |
|---|---|---|---|---|---|---|
| Théorie %<br>Trouvé % | 39,86<br>39,92 | 5,95<br>6,05 | 10,30<br>10,32 | 5,90<br>6,10 | 11,82<br>11,66 | 26,14<br>25,98 |

Exemple 4

Préparation du dichlorhydrate de 6-(β-acétylaminoéthylthio) 1,3-diamino benzène.

1ère étape

Synthèse du 6-(β-acétylaminoéthylthio) 3-nitro 1-acétylaminobenzène.

On dissout 20 g de potasse en poudre dans une solution de 71,4 g (0,6 mole) de β-acétylaminoéthylthiol dans 300 ml de diméthoxyéthane chauffé à 45 ° C.

Après refroidissement à 30 ° C on ajoute par portions, en 30 mn, 47,5 g (0,24 mole) de 2-acétylamino 4-nitrofluorobenzène.

On maintient la suspension à 30-35 ° C pendant 30 mn.

Le milieu réactionnel est versé dans 800 ml d'eau glacée. Le précipité cristallisé jaune est essoré, réempaté dans l'eau et séché sur anhydride phosphorique.

On obtient des cristaux jaunes (67,3 g) qui après recristallisation de l'éthanol fondent à 172 ° C et dont l'analyse élémentaire calculée pour $C_{12}H_{15}N_{3}0_{4}S$ est

| Analyse | C | H | N | O | S |
|---|---|---|---|---|---|
| Théorie %<br>Trouvé % | 48,48<br>48,69 | 5,09<br>5,16 | 14,13<br>14,00 | 21,52<br>21,74 | 10,78<br>10,80 |

2ème étape

Synthèse du 6-(β-acétylaminoéthylthio) 3-amino 1-acétylaminobenzène.

La réduction est effectuée selon le mode opératoire décrit pour l'exemple 2, (2ème étape). On obtient un composé cristallisé blanc dont le point de fusion est 148 ° C et dont l'analyse élémentaire calculée pour $C_{12}H_{17}N_{3}O_{2}S$ est :

| Analyse | C | H | N | O | S |
|---|---|---|---|---|---|
| Théorie %<br>Trouvé % | 53,91<br>53,95 | 6,41<br>6,39 | 15,72<br>15,90 | 11,97<br>12,08 | 11,99<br>11,95 |

3ème étape

La désacétylation du groupe amino en position 1 est effectuée en chauffant à 80 ° C pendant 5 heures une solution de 20 g (0,74 mole) du composé obtenu à l'étape précédente dans 300 ml d'une solution d'acide chlorhydrique normale.

On refroidit et neutralise avec une solution d'ammoniaque à 20 %.

Après extraction à l'acétate d'éthyle, séchage sur sulfate de sodium, filtration et évaporation à sec, l'huile obtenue et purifiée par passage sur colonne moyenne pression (gel de silice- éluant : mélange d'éthyle et d'heptane). Le dichlorydrate est préparé dans l'éthanol absolu chlorhydrique et précipité en diluant à l'éther éthylique. On essore et sèche sur potasse.

Les cristaux blancs (4,7 g) fondent avec décomposition à 201-204 ° C. Le spectre de masse est conforme au produit attendu.

## EXEMPLES DE PROCEDES DE TEINTURE

Exemples 1 à 4

### Composition A

| | |
|---|---|
| . Octyldodécanol vendu sous la dénomination EUTANOL D par la société HENKEL | 8,0 g |
| . Acide oléique | 20,0 g |
| . Lauryléthersulfate de monoéthanolamine vendu sous la dénomination SIPON LM 35 par la Société HENKEL | 3,0 g |
| . Alcool éthylique | 10,0 g |
| . Alcool benzylique | 10,0 g |
| . Alcool cétylstéarylique à 33 moles d'oxyde d'éthylène vendu sous la dénomination SIMULSOL GS par la Société SEPPIC | 2,4 g |
| . Acide éthylène diamine tétracétique | 0,2 g |
| . Solution aqueuse à 60 % de MA d'un polymère cationique présentant le motif récurrent suivant : 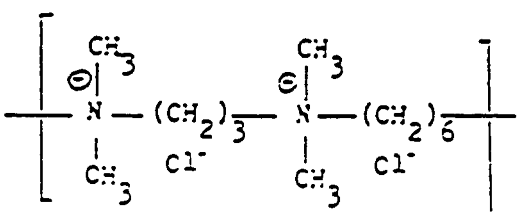 | 3,7 g MA |
| . Monoéthanolamine | 7,5 g |
| . Diéthanolamide d'acide linoléique vendu sous la dénomination COMPERLAN F par la Société HENKEL | 8,0 g |
| . Ammoniaque à 20 % de $NH_3$ | 10,2 g |
| . Métabisulfite de sodium en solution aqueuse à 35 % | 1,3 g |
| . Hydroquinone | 0,15 g |
| . Colorants | x g |
| . Eau déminéralisée qsp | 100,0 g |

### Composition B

Eau oxygénée à 20 volumes et à pH = 3,

### Protocole de teinture

La composition A est mélangée, poids pour poids, avec la composition B. Le mélange obtenu est appliqué sur des cheveux gris naturels à 90 % de blancs, permanentés ou non, pendant 30 mn. Les cheveux sont ensuite rincés, lavés au shampooing, puis rincés à nouveau et séchés.

| EXEMPLES | 1 | 2 | 3 | 4 |
|---|---|---|---|---|
| (en g) | | | | |
| <u>Composition A contenant</u> : | | | | |
| - 6-méthylthio 3-N-β-hydroxyéthylamino aniline, dichlorhydrate | 0,54 | | | |
| - 6-méthylthio 1-N-β-hydroxyéthylamino 3-aminobenzène | | 0,40 | | |
| - 6-méthylthio 1,3-di (β-hydroxyéthylamino) benzène, dichlorhydrate | | | 0,63 | |
| - 6-(β-acétylaminoéthylthio) 1,3-diaminobenzène, dichlorhydrate | | | | 0,60 |
| - Paraphénylènediamine | 0,22 | 0,22 | 0,22 | 0,22 |
| <u>Mélange poids pour poids de A et B</u> | | | | |
| pH du mélange | 10,2 | 10,3 | 10,5 | 10,3 |
| <u>Nuances obtenues</u> | | | | |
| . Sur cheveux gris naturels à 90 % de blancs | | gris bleuté | gris nacré | |
| . Sur cheveux gris naturels à 90 % de blancs permanentés | cendré nacré puissant | | | blond cendré |

Exemples 5 à 9

**Composition A**

| | |
|---|---|
| . Alcool oléique polyglycérolé à 2 moles de glycérol | 4,0 g |
| . Alcool oléique polyglycérolé à 4 moles de glycérol à 78 % de MA | 5,69 g MA |
| . Acide oléique | 3,0 g |
| . Amine oléique à 2 moles d'oxyde d'éthylène vendue sous la dénomination ETHOMEEN 012 par la Société AKZO | 7,0 g |
| . Laurylamino succinamate de diéthylaminopropyle, sel de sodium à 55% de MA | 3,0 g MA |
| . Alcool oléique | 5,0 g |
| . Diéthanolamide d'acide oléique | 12,0 g |
| . Propylèneglycol | 3,5 g |
| . Alcool éthylique | 7,0 g |
| . Dipropylèneglycol | 0,5 g |
| . Monométhyléther de propylèneglycol | 9,0 g |
| . Métabisulfite de sodium en solution aqueuse à 35 % | 0,455 g |
| . Acétate d'ammonium | 0,8 g |
| . Antioxydant, séquestrant qs | |
| . Parfum, conservateurs qs | |
| . Monoéthanolamine qsp pH 9,8 | |
| . Colorants | x g |
| . Eau déminéralisée qsp | 100,0 g |

**Composition B**

Elle est constituée par une solution d'eau oxygénée à 20 volumes dont le pH et ajusté entre 1 et 1,5 par de l'acide orthophosphorique.

**Protocole de teinture**

La composition A est mélangée, poids pour poids, avec la composition B d'eau oxygénée. Le mélange obtenu est appliqué sur des cheveux gris naturels à 90 % de blancs, permanentés ou non, pendant 30 minutes. Les cheveux sont ensuite rincés, lavés au shampooing, puis rincés à nouveau et séchés.

| EXEMPLES | 5 | 6 | 7 | 8 | 9 |
|---|---|---|---|---|---|
| (en g)<br>**Composition A contenant** : | | | | | |
| - 6-méthylthio 3-N-β-hydroxyéthylamino aniline, dichlorhydrate | 0,81 | | | | |
| - 6-méthylthio 1,3-diaminobenzène, dichlorhydrate | | 0,68 | | | |
| - 6-méthylthio 1-N-β-hydroxyéthylamino 3-aminobenzène | | | 0,59 | | |
| - 6-méthylthio 1,3-di (β-hydroxyéthylamino) benzène, dichlorhydrate | | | | 0,95 | |
| - 6-(β-acétylaminoéthylthio) 1,3-diaminobenzène, dichlorhydrate | | | | | 1,19 |
| - 2,6-diméthylparaphénylènediamine | 0,41 | 0,41 | 0,41 | 0,41 | 0,54 |
| **Mélange poids pour poids de A et B**<br>pH du mélange | 6,2 | 6,2 | 6,5 | 6,2 | 6,0 |
| **Nuances obtenues**<br>. Sur cheveux gris naturels à 90 % de blancs | | bleu puissant | | bleu cendré | bleu canard |
| . Sur cheveux gris naturels à 90 % de blancs permanentés | bleu cendré puissant | | bleu cendré | | |

## Revendications

1. Composition tinctoriale pour fibres kératiniques et en particulier pour cheveux humains, caractérisée par le fait qu'elle comprend, dans un milieu approprié pour la teinture, au moins un précurseur de colorant

d'oxydation de type ortho et/ou para et au moins, à titre de coupleur, une métaphénylènediamine soufrée de formule :

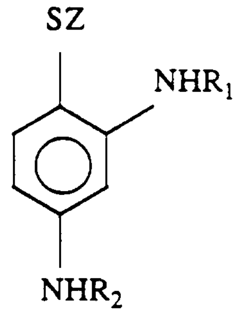

(I)

dans laquelle : Z représente un radical alkyle en $C_1$-$C_{18,}$ un radical aralkyle dans lequel le radical alkyle est en $C_1$-$C_6$, un radical monohydroxyalkyle en $C_1$-$C_6$ ou polyhydroxyalkyle $C_2$-$C_6$, un radical aryle, un radical aminoalkyle de formule :

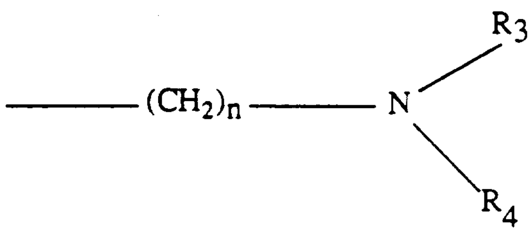

(II)

dans laquelle n est un nombre entier compris entre 1 et 6 inclus; $R_3$ et $R_4$, identiques aux différents, représentent un atome d'hydrogène ou un radical alkyle en $C_1$-$C_4$, hydroxyalkyle en $C_1$-$C_4$, acyle en $C_2$-$C_6$; $R_1$ et $R_2$, identiques ou différents, représentent un atome d'hydrogène, un radical alkyle en $C_1$-$C_6$, un radical monohydroxyalkyle en $C_1$-$C_6$, polyhydroxyalkyle en $C_2$-$C_6$, monocarbamylalkyle en $C_1$-$C_6$, dicarbamylalkyle en $C_1$-$C_6$, aminoalkyle en $C_1$-$C_6$, acyle en $C_2$-$C_6$, carbalcoxy en $C_2$-$C_6$, carbamyle ou monoalkyle en $C_1$-$C_6$ carbamyle, avec la condition que $R_1$ et $R_2$ ne désignent pas simultanément un atome d'hydrogène lorsque Z désigne alkyle ou hydroxyalkyle, ainsi que les sels d'acide correspondant aux composés de formule (I).

**2.** Composition tinctoriale selon la revendication 1, caractérisée par le fait que les précurseurs de colorants d'oxydation de type ortho ou para sont choisis parmi les paraphénylènediamines les paraaminophénols, les précurseurs hétérocycliques para dérivés de la pyridine ou de la pyrimidine, les orthoaminophénols et les bis-phénylalkylènediamines.

**3.** Composition selon la revendication 1 ou 2, caractérisée par le fait que dans le composé de formule (I), Z désigne un radical méthyle, éthyle, propyle, butyle, dodécyle, hexadécyle, benzyle, phényle, ou un radical mono ou polyhydroxyalkyle choisi parmi:

$-CH_2-CH_2-OH$, $-CH_2-CHOH-CH_2-OH$, $-CH_2-CHOH-CH_3$, aminoalkyle choisi parmi

$-CH_2-CH_2NH_2$, $-CH_2-CH_2-NHCH_3$ ,

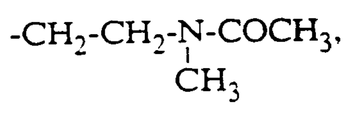

$-CH_2-CH_2-NH-COCH_3$ ;

$R_1$, $R_2$, $R_3$ ou $R_4$ désigne un radical acyle choisi parmi le formyle, l'acétyle et le propionyle.

4. Composition tinctoriale selon l'une quelconque des revendications 1 à 3 caractérisée en ce que les composés de formule (I) sont choisis parmi les composés suivants :
   - 6-méthylthio-di-($\beta$-hydroxyéthylamino) benzène,
   - 6-méthylthio 1-N-$\beta$-hydroxyéthylamino 3-aminobenzène.
   - 6-($\beta$-acétylaminoéthylthio) 1,3-diaminobenzène,
   - 6-méthylthio 3-N-$\beta$-hydroxyéthylamino aniline,
   - 6-($\beta$-acétylaminoéthylthio) 3-amino 1-acétylaminobenzène,
   - 6-méthylthio 3-acétylamino aniline,
   - 6-($\beta$-acétylaminoéthylthio) 3-acétylamino aniline et leurs sels d'addition avec un acide.

5. Composition selon l'une quelconque des revendications 1 à 4, caractérisée par le fait que les sels d'acide correspondants sont choisis parmi les chlorhydrates les sulfates, ou les bromhydrates.

6. Composition tinctoriale selon l'une quelconque des revendications 1 à 5, caractérisée en ce qu'elle contient outre les coupleurs de formule (I), d'autres coupleurs tels que des métadiphénols, des métaaminophénols, des métaphénylènediamines différentes de celles de formule (I), des métaacylaminophénols, des métauréidophénols, des métacarbalcoxyaminophénols, l'$\alpha$-naphtol, des dérivés indoliques, des coupleurs possédant un groupe méthylène actif.

7. Composition tinctoriale selon l'une quelconque des revendications 1 à 6, caractérisée en ce qu'elle contient des colorants directs.

8. Composition tinctoriale selon l'une quelconque des revendications 1 à 7, caractérisée en ce qu'elle contient 0, 05 à 3,5 % en poids du poids total de la composition d'au moins un composé de formule (I).

9. Composition tinctoriale selon l'une quelconque des revendications 1 à 8, caractérisée en ce qu'elle contient de 0,3 à 7 % en poids par rapport au poids total de la composition, de précurseur de colorant d'oxydation de type ortho et/ou para et de coupleurs.

10. Composition tinctoriale selon l'une quelconque des revendications 1 à 9, caractérisée en ce qu'elle contient des agents tensio-actifs cationiques, anioniques, non-ioniques, amphotères ou leurs mélanges, en des concentrations comprises entre 0,5 % et 55 % en poids par rapport au poids total de la composition ; des solvants organiques en des concentrations comprises entre 1 et 40 % en poids par rapport au poids total de la composition ; des agents épaississants en des concentrations comprises entre 0,1 et 5 % en, poids par rapport au poids total de la composition ; des agents antioxydants en des proportions comprises entre 0,05 et 1,5 % en poids par rapport au poids total de la composition ;

11. Composition tinctoriale selon l'une quelconque des revendications 1 à 10, caractérisée en ce qu'elle se présente sous forme de liquide, de crème, de gel ou tout autre forme appropriée, ou peut être conditionnée en flacon aérosol en présence d'un agent propulseur et former des mousses.

12. Procédé de teinture d'oxydation des fibres kératiniques et en particulier des cheveux humains, caractérisé en ce que l'on mélange, au moment de l'emploi, une composition tinctoriale pour fibres kératiniques et en particulier pour cheveux humains, comprenant, dans un milieu approprié pour la teinture, au moins un précurseur de colorant d'oxydation de type ortho et/ou para et au moins, à titre de coupleur, une métaphénylènediamine soufrée de formule :

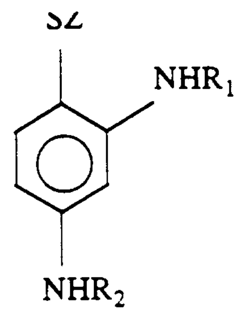

(I)

dans laquelle : Z représente un radical alkyle en $C_1$-$C_{18}$, un radical aralkyle dans lequel le radical alkyle est en $C_1$-$C_6$, un radical monohydroxyalkyle en $C_1$-$C_6$ ou polyhydroxyalkyle $C_2$-$C_6$, un radical aryle, un radical aminoalkyle de formule :

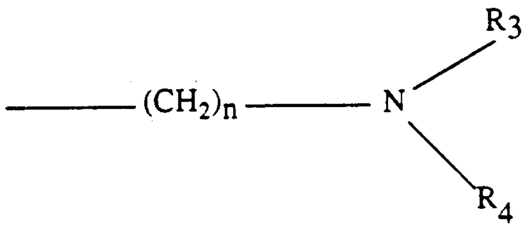

(II)

dans laquelle n est un nombre entier compris entre 1 et 6 inclus; $R_3$ et $R_4$, identiques aux différents, représentent un atome d'hydrogène ou un radical alkyle en $C_1$-$C_4$, hydroxyalkyle en $C_1$-$C_4$, acyle en $C_2$-$C_6$; $R_1$ et $R_2$, identiques ou différents, représentent un atome d'hydrogène, un radical alkyle en $C_1$-$C_6$, un radical monohydroxyalkyle en $C_1$-$C_6$, polyhydroxyalkyle en $C_2$-$C_6$, monocarbamylalkyle en $C_1$-$C_6$, dicarbamylalkyle en $C_1$-$C_6$, aminoalkyle en $C_1$-$C_6$, acyle en $C_2$-$C_6$, carbalcoxy en $C_2$-$C_6$, carbamyle ou monoalkyle en $C_1$-$C_6$ carbamyle, avec la condition que $R_1$ ou $R_2$ ne désignent pas simultanément un atome d'hydrogène lorsque Z désigne alkyle ou hydroxyalkyle, ainsi que les sels d'acide correspondant aux composés de formule (I), cette composition ne contenant pas d'ion métallique, avec une solution oxydante en une quantité suffisante pour développer la coloration la composition résultante ayant un pH variant entre 3 et 11, et on applique le mélange ainsi obtenu sur les fibres kératiniques en particulier les cheveux humains.

**13.** Procédé de teinture d'oxydation des fibres kératiniques et en particulier des cheveux humains selon la revendication 12, caractérisé en ce que l'on applique dans un premier temps le précurseur de colorant d'oxydation de type ortho et/ou para ou leur mélange sur les fibres kératiniques, dans un deuxième temps une composition contenant le coupleur de formule (I) tel que défini dans la revendication 1, et l'on développe la coloration à l'aide d'un agent oxydant présent dans ladite composition ou bien l'on applique cet agent oxydant sur les fibres kératiniques dans un troisième temps.

**14.** Procédé de teinture d'oxydation selon la revendication 12 ou 13, caractérisé par le fait qu'on laisse poser pendant 10 à 40 minutes, de préférence 15 à 30 minutes, on rince les cheveux, on les lave au shampooing, on les rince à nouveau et on les sèche.

**15.** Nouvelles métaphénylènediamines soufrées de formule générale :

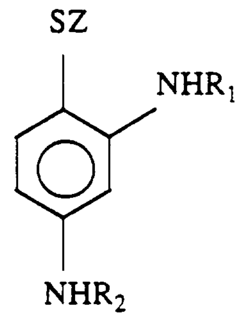

(I)

dans laquelle, Z désigne un radical alkyle en $C_1$-$C_{18}$, un radical aralkyle dans lequel le radical alkyle est en $C_1$-$C_6$, un radical monohydroxyalkyle en $C_1$-$C_6$ ou polyhydroxyalkyle en $C_2$-$C_6$, un radical aryle, un radical aminoalkyle de formule :

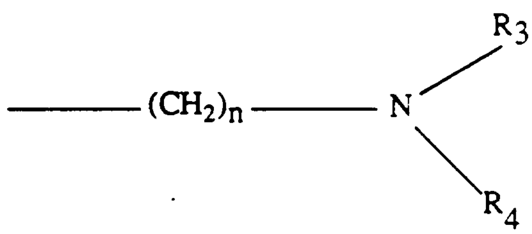

(II)

dans laquelle n est un nombre entier compris entre 1 et 6 inclus; $R_3$ et $R_4$, identiques ou différents, représentent un atome d'hydrogène un radical alkyle en $C_1$-$C_4$, hydroxyalkyle en $C_1$-$C_4$ ou acyle en $C_2$-$C_6$ ; $R_1$ et $R_2$, identiques ou différents, désignent un atome d'hydrogène, un radical alkyle en $C_1$-$C_6$, monohydroxyalkyle en $C_1$-$C_6$, polyhydroxyalkyle en $C_2$-$C_6$, monocarbamylalkyle en $C_1$-$C_6$, dicarbamylalkyle en $C_2$-$C_6$, aminoalkyle en $C_1$-$C_6$, acyle en $C_2$-$C_6$, carbalcoxy en $C_2$-$C_6$, carbamyle ou monoalkyle en $C_1$-$C_6$ carbamyle, à la condition que $R_1$ et $R_2$ ne désignent pas simultanément un atome d'hydrogène lorsque Z désigne un radical alkyle ou hydroxyalkyle ainsi que les sels d'acide correspondants aux composés de formule (I).

**16.** Nouvelles métaphénylènediamines soufrées selon la revendication 15, caractérisées en ce qu'elles sont choisies parmi

- le 6-méthylthio 1,3-di-($\beta$-hydroxyéthylamino)benzène,
- le 6-méthylthio 1-N-$\beta$-hydroxyéthylamino 3-aminobenzène,
- le 6-($\beta$-acétylaminoéthylthio) 1,3-diaminobenzène,
- la 6-méthylthio 3-N-$\beta$-hydroxyéthylamino aniline,
- le 6-($\beta$-acétylaminoéthylthio)3-amino 1-acétylaminobenzène,
- la 6-méthylthio 3-acétylamino aniline,
- la 6-($\beta$-acétylaminoéthylthio) 3-acétylamino aniline, et leurs sels d'addition avec un acide.

**17.** Utilisation des nouvelles métaphénylènediamines soufrées selon la revendication 15 ou 16 dans des compositions tinctoriales pour fibres kératiniques.

## Claims

**1.** Dyeing composition for keratinous fibers and in particular human hair, characterized by the fact that it comprises, in a medium suitable for dyeing, at least one ortho and/or para type oxidation dye precursor and at least, as coupler, one sulfur-containing meta-phenylenediamine of formula:

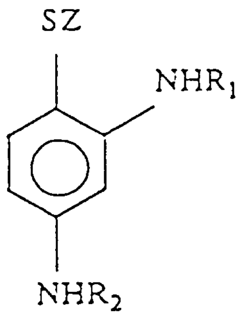

(I)

in which: Z represents a $C_1$-$C_{18}$ alkyl radical, an aralkyl radical in which the alkyl radical corresponds to $C_1$-$C_6$, a monohydroxy($C_1$-$C_6$ alkyl) or polyhydroxy($C_2$-$C_6$ alkyl) radical, an aryl radical, an aminoalkyl radical of formula:

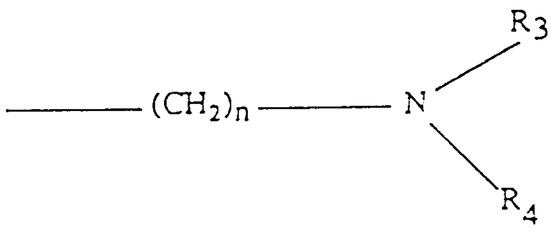

(II)

in which n is an integer between 1 and 6 inclusive, $R_3$ and $R_4$, which are identical or different, represent a hydrogen atom or $C_1$-$C_4$ alkyl, hydroxy($C_1$-$C_4$ alkyl) or $C_2$-$C_6$ acyl radical; $R_1$ and $R_2$, which are identical or different, represent a hydrogen atom, a $C_1$-$C_6$ alkyl radical, a monohydroxy($C_1$-$C_6$ alkyl), polyhydroxy($C_2$-$C_6$ alkyl), monocarbamyl($C_1$-$C_6$ alkyl), dicarbamyl($C_1$-$C_6$ alkyl), amino($C_1$-$C_6$ alkyl), $C_2$-$C_6$ acyl, $C_2$-$C_6$ carbalkoxy, carbamyl or mono($C_1$-$C_6$ alkyl)carbamyl radical, with the proviso that $R_1$ and $R_2$ do not simultaneously designate a hydrogen atom when Z designates an alkyl or hydroxyalkyl radical, as well as the acid salts corresponding to the compounds of formula (I).

**2.** Dyeing composition according to claim 1, characterized by the fact that the ortho or para type oxidation dye precursors are chosen from para-phenylenediamines, para-aminophenols, para heterocyclic precursors derived from pyridine or pyrimidine, ortho-aminophenols and bis-phenylalkylenediamines.

**3.** Composition according to claim 1 or 2, characterized by the fact that in the compound of formula (I), Z designates a methyl, ethyl, propyl, butyl, dodecyl, hexadecyl, benzyl or phenyl radical or a mono- or polyhydroxyalkyl radical chosen from:
$-CH_2$-$CH_2$-OH, $-CH_2$-CHOH-$CH_2$-OH, $-CH_2$-CHOH-$CH_3$,
or an aminoalkyl radical chosen from
$-CH_2$-$CH_2NH_2$, $-CH_2$-$CH_2$-$NHCH_3$,

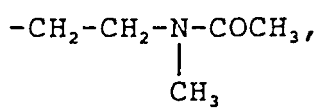

$-CH_2$-$CH_2$-NH-$COCH_3$;
$R_1$, $R_2$, $R_3$ or $R_4$ designates an acyl radical chosen from formyl, acetyl and propionyl.

**4.** Dyeing composition according to any one of claims 1 to 3, characterized in that the compounds of formula (I) are chosen from the following compounds:
- 6-methylthio-di-(β-hydroxyethylamino)benzene,
- 6-methylthio-1-N-β-hydroxyethylamino-3-aminobenzene,

- 6-(β-acetylaminoethylthio)-1,3-diaminobenzene,
- 6-methylthio-3-N-β-hydroxyethylaminoaniline,
- 6-(β-acetylaminoethylthio)-3-amino-1-acetylaminobenzene,
- 6-methylthio-3-acetylaminoaniline,
- 6-(β-acetylaminoethylthio)-3-acetylaminoaniline and their addition salts with an acid.

**5.** Composition according to any one of claims 1 to 4, characterized by the fact that the corresponding salts with an acid are chosen from hydrochlorides, sulfates or hydrobromides.

**6.** Dyeing composition according to any one of claims 1 to 5, characterized in that it contains, in addition to the couplers of formula (I), other couplers such as meta-diphenols, meta-aminophenols, meta-phenylenediamines different from those of formula (I), meta-acylaminophenols, meta-ureidophenols, meta-carbalkoxyaminophenols, α-naphthol, indole derivatives, couplers possessing an active methylene group.

**7.** Dyeing composition according to any one of claims 1 to 6, characterized in that it contains direct dyes.

**8.** Dyeing composition according to any one of claims 1 to 7, characterized in that it contains 0.05 to 3.5% by weight of the total weight of the composition of at least one compound of formula (I).

**9.** Dyeing composition according to any one of claims 1 to 8, characterized in that it contains 0.3 to 7% by weight relative to the total weight of the composition, of ortho and/or para type oxidation dye precursor and couplers.

**10.** Dyeing composition according to any one of claims 1 to 9, characterized in that it contains cationic, anionic, non-ionic or amphoteric surface-active agents or mixtures thereof, in concentrations of between 0.5% and 55% by weight relative to the total weight of the composition; organic solvents in concentrations of between 1 and 40% by weight relative to the total weight of the composition; thickening agents in concentrations of between 0.1 and 5% by weight relative to the total weight of the composition; antioxidants in proportions of between 0.05 and 1.5% by weight relative to the total weight of the composition;

**11.** Dyeing composition according to any one of claims 1 to 10, characterized in that it is provided in the form of a liquid, cream or gel or any other appropriate form, or may be packaged in aerosol bottle in the presence of a coupling agent and may form foams.

**12.** Process for the oxidative dyeing of keratinous fibers and in particular human hair, characterized in that a dyeing composition for keratinous fibers and in particular for human hair, comprising, in a medium suitable for dyeing, at least one ortho and/or para type oxidation dye precursor and at least, as coupler, one sulfur-containing meta-phenylenediamine of formula:

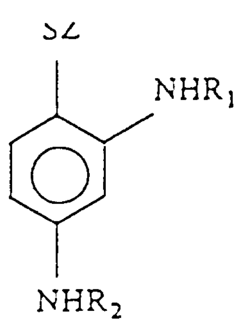

(I)

in which: Z represents a $C_1$-$C_{18}$ alkyl radical, an aralkyl radical in which the alkyl radical corresponds to $C_1$-$C_6$, a monohydroxy($C_1$-$C_6$ alkyl) or polyhydroxy($C_2$-$C_6$ alkyl) radical, an aryl radical, an aminoalkyl radical of formula:

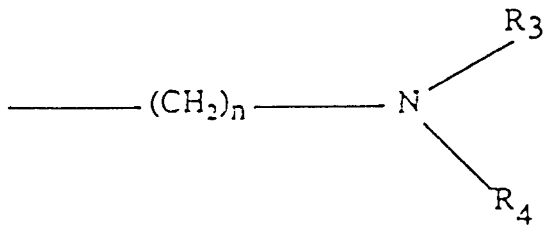

(II)

in which n is an integer between 1 and 6 inclusive, $R_3$ and $R_4$, which are identical to the [sic] different, represent a hydrogen atom or $C_1$-$C_4$ alkyl, hydroxy ($C_1$-$C_4$ alkyl) or $C_2$-$C_6$ acyl radical, $R_1$ and $R_2$, which are identical or different, represent a hydrogen atom, a $C_1$-$C_6$ alkyl radical, a monohydroxy($C_1$-$C_6$ alkyl), polyhydroxy($C_2$-$C_6$ alkyl), monocarbamyl($C_1$-$C_6$ alkyl), dicarbamyl($C_1$-$C_6$ alkyl), amino($C_1$-$C_6$ alkyl), $C_2$-$C_6$ acyl, $C_2$-$C_6$ carbalkoxy, carbamyl or mono($C_1$-$C_6$ alkyl)carbamyl radical, with the proviso that $R_1$ and $R_2$ do not simultaneously designate a hydrogen atom when Z designates alkyl or hydroxyalkyl, as well as the acid salts corresponding to the compounds of formula (I), this composition not containing metal ions, is mixed, at the time of use, with an oxidizing solution in a sufficient quantity so as to develop the color, the resulting composition having a pH varying between 3 and 11, and the mixture thus obtained is applied to the keratinous fibers, in particular human hair.

**13.** Process for the oxidative dyeing of keratinous fibers and in particular human hair according to claim 12, characterized in that in a first phase, the ortho and/or para type oxidation dye precursor or mixture thereof is applied to the keratinous fibers, in a second phase, a composition containing the coupler of formula (I) as defined in claim 1, and the color is developed by means of an oxidizing agent present in the said composition or alternatively this oxidizing agent is applied to the keratinous fibers in a third phase.

**14.** Process for oxidative dyeing according to claim 12 or 13, characterized by the fact that the products are allowed to act for 10 to 40 minutes, preferably 15 to 30 minutes, the hair is rinsed, it is washed with shampoo, it is rinsed again and it is dried.

**15.** New sulfur-containing meta-phenylenediamines of general formula:

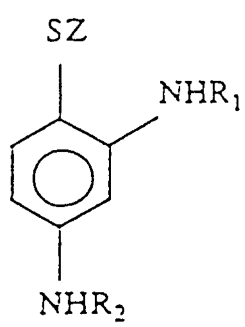

(I)

in which: Z represents a $C_1$-$C_{18}$ alkyl radical, an aralkyl radical in which the alkyl radical corresponds to $C_1$-$C_6$, a monohydroxy($C_1$-$C_6$ alkyl) or polyhydroxy($C_2$-$C_6$ alkyl) radical, an aryl radical, an aminoalkyl radical of formula:

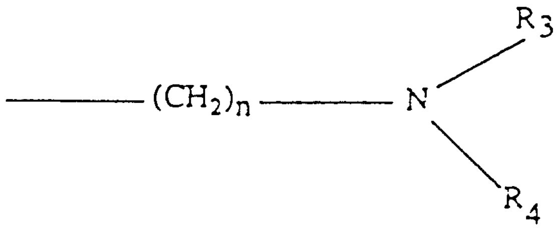

(II)

in which n is an integer between 1 and 6 inclusive, $R_3$ and $R_4$, which are identical or different, represent a hydrogen atom or $C_1$-$C_4$ alkyl, hydroxy($C_1$-$C_4$ alkyl) or $C_2$-$C_6$ acyl radical, $R_1$ and $R_2$, which are identical or different, represent a hydrogen atom, a $C_1$-$C_6$ alkyl radical, a monohydroxy($C_1$-$C_6$ alkyl), polyhydroxy($C_2$-$C_6$ alkyl), monocarbamyl($C_1$-$C_6$ alkyl), dicarbamyl($C_2$-$C_6$ alkyl), amino($C_1$-$C_6$ alkyl), $C_2$-$C_6$ acyl, $C_2$-$C_6$ carbalkoxy, carbamyl or mono($C_1$-$C_6$ alkyl)carbamyl radical, with the proviso that $R_1$ and $R_2$ do not simultaneously designate a hydrogen atom when Z designates an alkyl or hydroxyalkyl radical, as well as the acid salts corresponding to the compounds of formula (I).

**16.** New sulfur-containing meta-phenylenediamines according to claim 15, characterized in that they are chosen from:

- 6-methylthio-1,3-di-(β-hydroxyethylamino)benzene,
- 6-methylthio-1-N-(β-hydroxyethylamino-3-aminobenzene,
- 6-(β-acetylaminoethylthio)-1,3-diaminobenzene,
- 6-methylthio-3-N-β-hydroxyethylaminoaniline,
- 6-(β-acetylaminoethylthio)-3-amino-1-acetylaminobenzene,
- 6-methylthio-3-acetylaminoaniline,
- 6-(β-acetylaminoethylthio)-3-acetylaminoaniline, and their addition salts with an acid.

**17.** Use of the new sulfur-containing meta-phenylenediamines according to claim 15 or 16 in dyeing compositions for keratinous fibers.

**Patentansprüche**

**1.** Färbezusammensetzung für keratinische Fasern und fürinsbesondere menschliche Haare, dadurch **gekennzeichnet**, daß sie, in einem zur Färbung geeigneten Milieu, mindestens eine Oxidationsfarbstoff-Vorstufe vom ortho- und/oder para-Typ und mindestens, als Kuppler, ein sulfuriertes m-Phenylendiamin der Formel:

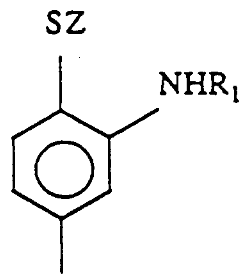

(I)

worin Z einen $C_{1-18}$-Alkylrest, Aralkylrest, in welchem der Alkylrest ein $C_{1-6}$-Rest ist, einen Monohydroxy-$C_{1-6}$-alkyl- oder Polyhydroxy $C_{2-6}$-alkylrest, einen Arylrest, einen Aminoalkylrest der Formel:

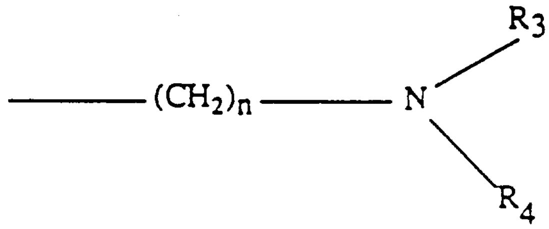

(II)

in welchem n eine ganze Zahl von 1 bis 6 ist und $R_3$ und $R_4$, gleich oder verschieden, ein Wasserstoffatom oder einen $C_{1-4}$-Alkyl-,Hydroxy-$C_{1-4}$-alkyl- oder $C_{2-6}$-Acylrest darstellen, und worin $R_1$ und $R_2$, gleich oder verschieden, ein Wasserstoffatom, einen $C_{1-6}$-Alkyl-, Monohydroxy-$C_{1-6}$-alkyl-, Polyhydroxy-$C_{2-6}$-alkyl-, Monocarbamyl-$C_{1-6}$-alkyl-, Dicarbamyl-$C_{1-6}$-alkyl-, Amino-$C_{1-6}$-alkyl-, $C_{2-6}$-Acyl-, $C_{2-6}$-Carbalkoxy-, Carbamyl- oder $C_{1-6}$-Monoalkylcarbamylrest darstellen, mit der Maßgabe, daß $R_1$ und $R_2$ nicht gleichzeitig ein Wasserstoffatom bedeuten, wenn Z einen Alkyl- oder Hydroxyalkylrest bedeutet, sowie die den Verbindungen der Formel (I) entsprechenden Säuresalze enthält.

**2.** Färbezusammensetzung gemäß Anspruch 1,
dadurch **gekennzeichnet**, daß
die Oxidationsfarbstoff-Vorstufen vom ortho- oder para-Typ aus p-Phenylendiaminen, p-Aminophenolen, von Pyridin oder Pyrimidin abgeleiteten heterozyklischen Vorstufen vom para-Typ, o-Aminophenolen und Bisphenylalkylendiaminen ausgewählt sind.

**3.** Zusammensetzung gemäß Anspruch 1 oder 2,
dadurch **gekennzeichnet**, daß
in der Verbindung der Formel (I) Z einen Methyl-, Ethyl-, Propyl-, Butyl-, Dodecyl-, Hexadecyl-, Benzyl-, Phenyl- oder einen Mono- oder Polyhydroxyalkylrest, ausgewählt aus: $-CH_2-CH_2OH$, $-CH_2-CHOH-CH_2-OH$, $-CH_2-CHOH-CH_3$
oder einen Aminoalkylrest bedeutet, ausgewählt aus: $-CH_2-CH_2-NH_2$, $-CH_2-CH_2-N-CH_3-COCH_3$, $-CH_2-CH_2-NHCH_3$, $-CH_2-CH_2-NHCOCH_3$,
und daß $R_1$, $R_2$, $R_3$ oder $R_4$ einen Acylrest aus dem Formyl-, Acetyl- und Propionylrest bedeutet.

**4.** Färbezusammensetzung gemäß jedem der Ansprüche 1 bis 3, dadurch **gekennzeichnet**, daß
die Verbindungen der Formel (I) aus den folgenden Verbindungen ausgewählt sind:
- 6-Methylthio-1,3-di($\beta$-hydroxyethylamino)benzol
- 6-Methylthio-1-N-$\beta$-hydroxyethylamino-3-aminobenzol
- 6-($\beta$-Acetylaminoethylthio)-1,3-diaminobenzol
- 6-Methylthio-3-N-$\beta$-hydroxyethylaminoanilin
- 6-Methylthio-3-acetylaminoanilin
- 6-($\beta$-Acetylaminoethylthio)-3-acetylaminoanilin
- 6-($\beta$-Acetylaminoethylthio)-3-amino-1-acetylaminobenzol

sowie aus deren Additionssalzen mit einer Säure.

**5.** Zusammensetzung gemäß jedem der Ansprüche 1 bis 4, dadurch **gekennzeichnet**, daß
die entsprechenden Säuresalze aus Hydrochloriden, Sulfaten oder Hydrobromiden ausgewählt sind.

**6.** Färbezusammensetzung gemäß jedem der Ansprüche 1 bis 5, dadurch **gekennzeichnet**, daß
sie ausser den Kupplern der Formel (I) weitere Kuppler wie m-Diphenole, m-Aminophenole, von denjenigen der Formel (I) verschiedene, m-Phenylendiamine, m-Acylaminophenole, m-Ureidophenole, m-Carbalcoxyaminophenle, $\alpha$-Naphthol, Indolderivate und Kuppler mit einer aktiven Methylengruppe enthält.

**7.** Färbezusammensetzung gemäß jedem der Ansprüche 1 bis 6, dadurch **gekennzeichnet**, daß
sie Direktfarbstoffe enthält.

**8.** Färbezusammensetzung gemäß jedem der Ansprüche 1 bis 7, dadurch **gekennzeichnet**, daß
sie 0,05 bis 3,5 Gew.% des Gesamtgewichts der Zusammensetzung mindestens einer Verbindung der Formel (I) enthält.

9. Färbezusammensetzung gemaß jedem der Ansprüche 1 bis 8, dadurch **gekennzeichnet**, daß sie 0,3 bis 7 Gew.%, bezogen auf das Gesamtgewicht der Zusammensetzung, Oxidationsfarbstoff-Vorstufe vom ortho- und/oder para-Typ und Kuppler enthält.

10. Färbezusammensetzung gemäß jedem der Ansprüche 1 bis 9, dadurch **gekennzeichnet**, daß sie kationische, anionische, nicht-ionische oder amphotere oberflächenaktive Mittel oder deren Mischungen in Konzentrationen von 0,5 bis 55 Gew.%, bezogen auf das Gesamtgewicht der Zusammensetzung, organische Lösungsmittel in Konzentrationen von 1 bis 40 Gew.%, bezogen auf das Gesamtgewicht der Zusammensetzung, Verdickungsmittel in Konzentrationen von 0,1 bis 5 Gew.%, bezogen auf das Gesamtgewicht der Zusammensetzung, und Antioxidantien in Mengenanteilen von 0,05 bis 1,5 Gew.%, bezogen auf das Gesamtgewicht der Zusammensetzung, enthält.

11. Färbezusammensetzung gemäß jedem der Ansprüche 1 bis 10, dadurch **gekennzeichnet**, daß sie in Form einer Flüssigkeit, Creme, eines Gels oder in jeder weiteren geeigneten Form vorliegt oder als Aerosol-Fläschchen in Gegenwart eines Treibmittels zubereitet sein und Schäume bilden kann.

12. Verfahren zur Oxidationsfärbung keratinischer Fasern und insbesondere menschlicher Haare, dadurch **gekennzeichnet**, daß man, zum Zeitpunkt der Anwendung, eine Färbezusammensetzung für keratinische Fasern und insbesondere für menschliche Haare, die, in einem zur Färbung geeigneten Milieu, mindestens eine Oxidationsfarbstoff-Vorstufe vom ortho- und/oder para-Typ und mindestens, als Kuppler, ein sulfuriertes m-Phenylendiamin der Formel:

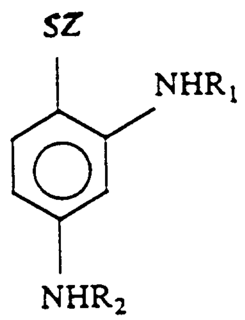

(I)

worin Z einen $C_{1-18}$-Alkylrest, Aralkylrest, in welchem der Alkylrest ein $C_{1-6}$-Rest ist, einen Monohydroxy-$C_{1-6}$-alkyl- oder Polyhydroxy-$C_{2-6}$-alkylrest, einen Arylrest, einen Aminoalkylrest der Formel:

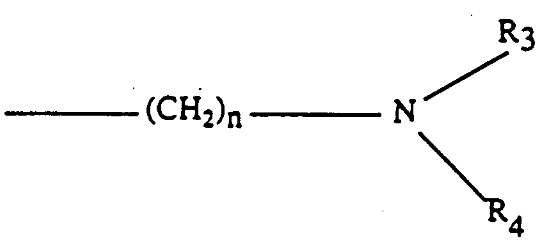

(II)

in welchem n eine ganze Zahl von 1 bis 6 ist und $R_3$ und $R_4$, gleich oder verschieden, ein Wasserstoffatom oder einen $C_{1-4}$-Alkyl-,Hydroxy-$C_{1-4}$-alkyl- oder $C_{2-6}$-Acylrest darstellen, und worin $R_1$ und $R_2$, gleich oder verschieden, ein Wasserstoffatom, einen $C_{1-6}$-Alkyl-, Monohydroxy-, $C_{1-6}$-Alkyl-, Polyhydroxy-, $C_{2-6}$-Alkyl-, Monocarbamyl-$C_{1-6}$-alkyl-, Dicarbamyl-$C_{1-6}$-alkyl-, Amino-$C_{1-6}$-alkyl-, $C_{2-6}$-Acyl-, $C_{2-6}$-Carbalkoxy-, Carbamyl- oder $C_{1-6}$-Monoalkylcarbamylrest darstellen, mit der Maßgabe, daß $R_1$ und $R_2$ nicht gleichzeitig ein Wasserstoffatom bedeuten, wenn Z einen Alkyl- oder Hydroxyalkylrest bedeutet, sowie die den Verbindungen der Formel (I) entsprechenden Säuresalze enthält, wobei diese Zusammensetzung kein Metallion aufweist, mit einer oxidierenden Lösung in einer zur Entwicklung der Färbung ausreichenden Menge vermischt, wobei die sich ergebende Zusammensetzung einen pH von 3 bis 11 aufweist, und daß man die so erhaltene Mischung auf die keratinischen Fasern, insbesondere die menschlichen Haare, aufbringt.

**13.** Verfahren zur Oxidationsfärbung keratinischer Fasern und insbesondere menschlicher Haare gemäß Anspruch 12,
dadurch **gekennzeichnet**, daß
man zu einem ersten Zeitpunkt die Oxidationsfarbstoff-Vorstufe vom ortho- und/oder para-Typ oder deren Mischung auf die keratinischen Fasern, zu einem zweiten Zeitpunkt eine den Kuppler der in Anspruch 1 definierten Formel (I) enthaltende Zusammensetzung aufbringt und man die Färbung mittels eines oxidierenden Agens, das in der genannten Zusammensetzung vorhanden ist, entwickelt, oder daß man dieses oxidierende Agens auf die keratinischen Fasern auch zu einem dritten Zeitpunkt aufbringt.

**14.** Verfahren zur Oxidationsfärbung gemäß Anspruch 12 oder 13,
dadurch **gekennzeichnet**, daß
man die Mischung 10 bis 40 und vorzugsweise 15 bis 30 Minuten lang verweilen läßt, die Haare spült, sie unter Schamponieren wäscht, erneut spült und dann trocknet.

**15.** Neue sulfurierte m-Phenylendiamine der allgemeinen Formel :

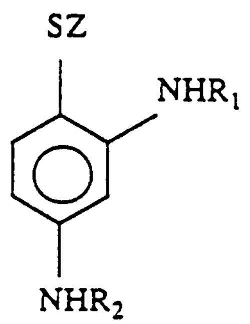

(I)

worin Z einen $C_{1-18}$-Alkylrest, Aralkylrest, in welchem der Alkylrest ein $C_{1-6}$-Rest ist, einen Monohydroxy-$C_{1-6}$-alkyl- oder Polyhydroxy-$C_{2-6}$-alkylrest, einen Arylrest, einen Aminoalkylrest der Formel:

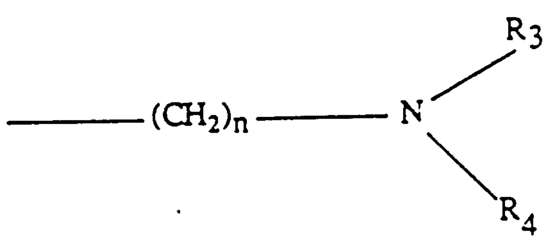

(II)

in welchem n eine ganze Zahl von 1 bis 6 ist und $R_3$ und $R_4$, gleich oder verschieden, ein Wasserstoffatom oder einen $C_{1-4}$-Alkyl-,Hydroxy-$C_{1-4}$-alkyl- oder $C_{2-6}$-Acylrest darstellen, und worin $R_1$ und $R_2$, gleich oder verschieden, ein Wasserstoffatom, einen $C_{1-6}$-Alkyl-, Monohydroxy-$C_{1-6}$-alkyl-, Polyhydroxy-$C_{2-6}$-alkyl-, Monocarbamyl-$C_{1-6}$-alkyl-, Dicarbamyl-$C_{1-6}$-alkyl-, Amino-$C_{1-6}$-alkyl-, $C_{2-6}$-Acyl-, $C_{2-6}$-Carbalkoxy-, Carbamyl- oder $C_{1-6}$-Monoalkylcarbamylrest darstellen, mit der Maßgabe, daß $R_1$ und $R_2$ nicht gleichzeitig ein Wasserstoffatom bedeuten, wenn Z einen Alkyl- oder Hydroxyalkylrest bedeutet,
sowie die den Verbindungen der Formel (I) entsprechenden Säuresalze.

**16.** Neue sulfurierte m-Phenylendiamine gemäß Anspruch 15, dadurch **gekennzeichnet**, daß
sie aus den folgenden Verbindungen ausgewählt sind:
- 6-Methylthio-1,3-di($\beta$-hydroxyethylamino)benzol

- 6-Methylthio-1-N-β-hydroxyethylamino-3-aminobenzol
- 6-(β-Acetylaminoethylthio)-1,3-diaminobenzol
- 6-Methylthio-3-N-β-hydroxyethylaminoanilin
- 6-Methylthio-3-acetylaminoanilin
- 6-(β-Acetylaminoethylthio)-3-acetylaminoanilin
- 6-(β-Acetylaminoethylthio)-3-amino-1-acetylaminobenzol

sowie aus den mit einer Säure hergestellten Additionssalzen ausgewählt sind.

**17.** Verwendung der neuen sulfurierten m-Phenylendiamine gemäß Anspruch 15 oder 16 in Färbezusammensetzungen für keratinische Fasern.